# EUROPÄISCHE PATENTSCHRIFT

(11) **EP 1 723 944 B1**
(45) Veröffentlichungstag und Bekanntmachung des Hinweises auf die Patenterteilung: **04.04.2007**
(21) Anmeldenummer: 05003253.1
(22) Anmeldetag: 16.02.2005
(51) Int. Cl.: A61K 8/35, A61K 8/37, A61K 8/49, A61Q 17/04

(54) **Kosmetische und dermatologische Lichtschutzformulierungen mit einem Gehalt an chemischen UV-Filtern und Triheptanonin**
Cosmetic and dermatological sunscreens comprising chemical UV-screens and glyceryl triheptanoate
Ecrans solaires cosmétiques et dermatologiques comprenant des filtres UV chimiques et du triheptanoate de glycéryl

(43) Veröffentlichungstag der Anmeldung: 22.11.2006
(73) Patentinhaber: Dr. Straetmans Chemische Produktion GmbH, 22143 Hamburg (DE)
(72) Erfinder: Straetmans, Udo Dr., 22143 Hamburg (DE); Jänichen, Jan Dr., 22143 Hamburg (DE); Petersen, Wildfried Dr., 22143 Hamburg (DE)
(74) Vertreter: Fleck, Thomas

(56) Entgegenhaltungen:
- US-A1- 2004 057 914
- US-A1- 2004 059 119
- US-A1- 2004 091 434
- US-B1- 6 372 204

## Beschreibung

### Technischer Hintergrund der Erfindung

Dem Schutz der menschlichen Haut vor schädigenden UV-Strahlen ist nicht nur durch die zunehmende Ausdünnung der Ozonschicht in einigen Teilen der Erde sondern auch durch das Freizeitverhalten des Menschen in den vergangenen Jahren eine zunehmende Bedeutung zugewachsen. Während der sehr kurzwellige UV-Bereich von < 290 nm normalerweise von der atmosphärischen Ozonschicht absorbiert wird, gelangen die längerwelligen UV-B Strahlen mit einer Wellenlänge von 290 - 320 nm sowie die UV-A Strahlen mit einer Wellenlänge von 320 - 400 nm relativ ungehindert an die Erdoberfläche und können je nach Einwirkungsdauer und -frequenz auf der Haut temporäre aber auch bleibende Schädigungen hervorrufen.

So führt bereits eine kurzzeitige Einwirkung von UV-Strahlung von ca. 310 nm rasch zu einem schmerzhaften Sonnenbrand während eine längere Einwirkung langwelliger UV-A-Strahlung in den tieferen Hautschichten phototoxische Reaktionen hervorrufen kann und über eine Schädigung des Bindegewebes die Haut schneller altern lässt. Jedoch kann nicht nur das Erscheinungsbild der Haut unter extensiver UV-Einwirkung leiden, auch maligne Zellveränderungen, verbunden mit der Ausbildung von Melanomen, werden UV-induzierten Schädigungen des genetischen Materials der Hautzellen zugeschrieben.

Vor diesem Hintergrund stellen chemische Verbindungen, die in der Lage sind, UV-Strahlen auf der Hautoberfläche zu absorbieren oder zu reflektieren, eine wichtige Substanzklasse für dermatologische und kosmetische Produkte dar und finden heutzutage nicht nur in Sonnenschutzprodukten sondern auch in Hautpflegeprodukten des Tagesbedarfs ein breite Anwendung.

Die Funktionsweise chemischer UV-Filter basiert darauf, dass diese in der Lage sind, energiereiche UV-Strahlung zu absorbieren und dabei in einen angeregten Zustand übergehen. Aus diesem relaxieren die angeregten Spezies anschließend unter inkrementärer Abgabe der aufgenommenen Energie wieder in ihren Grundzustand.

Anorganische Pigmente wie TiO₂ oder ZnO hingegen reflektieren die UV-Strahlung und verhindern auf diese Weise deren Eindringen in die Haut.

Strukturell gesehen gehören die chemischen UV-Filter zur Verbindungsklasse der Aromaten, deren UV-Absorptionsspektrum durch Substituenten und/oder Heteroatome in den geforderten UV-Bereich verschoben wurde. Diese Verbindungen besitzen bis auf wenige Ausnahmen nur eine geringe Wasserlöslichkeit und zeichnen sich häufig durch sehr ausgeprägte Kristallisationseigenschaften aus. Letztere Eigenschaft hat häufig auch eine begrenzte Öllöslichkeit zur Folge, was die Einsatzkonzentration chemischer UV-Filter und damit den Lichtschutzfaktor der betreffenden Formulierung limitiert.

Da die Absorptionsmaxima der chemischen UV-Filter von deren Struktur abhängen und nicht den gesamten Bereich schädlicher UV-A- und UV-B-Strahlung abdecken, werden in der Regel Gemische von UV-Filtern mit unterschiedlichen Absorptionsmaxima in Sonnenschutzprodukten eingesetzt, um einen möglichst effektiven Breitbadschutz der Haut vor den schädlichen Effekten der UV-Strahlung zu gewährleisten.

Vorraussetzung für eine optimale Wirkung beider - der chemischen UV-Filter und der anorganischen Pigmente - ist aber auch ihre homogene Verteilung auf der Hautoberfläche sowie eine geringe Penetrationsneigung in die Haut. Letztere ist insbesondere für die chemischen Filter von großer Bedeutung, um Reaktionen der angeregten Spezies auf der Zelloberfläche von Hautzellen zu verhindern.

### Gegenstand der vorliegenden Erfindung

Geeignete Lösungsmittel für Sonnenschutzprodukte bilden idealerweise Ölkomponenten, die mehrere Eigenschaften in sich vereinen. So sollten sie:
- Lichtschutzfilter für verschiedene UV-Bereiche gut und stabil zu lösen
- anorganische Pigmente stabil zu dispergieren
- zum Zwecke einer homogenen Verteilung der Lichtschutzfaktoren auf der Haut über ein gutes Spreitvermögen verfügen
- nur eine geringe Pentrationsfähigkeit in die Haut besitzen.

Vorteilhafte UV-Filter, die sich durch gute Lichtschutzeigenschaften auszeichnen und die in der jüngsten Vergangenheit breiten Einsatz gefunden haben sind z.B. der UV-B-Filter 2,4,6-Trianilino-p-(carbo-2'-ethylhexyl-1'oxy)-1,3,5-triazin (INCI: **Ethylhexyl Triazon),** der von der BASF unter dem Handelsnamen Uvinul T 150 vertrieben wird, der UV-A-Filter 1-(4-tert-butylphenyl)-3-(4-methoxyphenyl)-propan-1,3-dion **(INCI: Butyl Methoxydibenzoylmethan),** welcher z.B. unter dem Handelsnamen Parsol 1789 von der Fa. DSM angeboten wird oder das Bis-Octyloxyphenol-methoxyphenyltriazin **(INCI: Bis-Ethylhexyloxyphenol Methoxyphenyl Triazine)**, ein Breitbandfilter, der z.B. unter dem Handelsnamen Tinosorb S von der Fa. Ciba Geigy (EP0775698 B1) angeboten wird. Als Nachteil dieser genannten UV-Filter ist jedoch ihre geringe Löslichkeit in zahlreichen, in der Kosmetik üblicherweise eingesetzten Ölkomponenten und ihre ausgeprägte Kristallisationsneigung zu nennen. Triglyceride werden gewöhlicherweise nicht als Lösungsmittel für chemische UV-Filter in Betracht gezogen, da diese nur eine sehr begrenzte Fähigkeit besitzen jene UV-Filter zu lösen. Vielmehr finden sich Lösungsmittel, die hinsichtlich dieser UV-Filter dem Stand der Technik entsprechen, beispielsweise in der Gruppe von Estern aromatischer Carbonsäuren (z.B. C12-15 Alkylbenzoat), die z.B. unter dem Namen Finsolv TN angeboten werden oder mittelkettiger Ester des Butylenglykols (Butylene Glycol Dicaprylate/Dicaprate), welcher unter dem Handelsnamen Dermofeel® BGC von der Dr. Straetmans GmbH in höherer Preislage angeboten wird. Beide Produkte basieren zumindest teilweise auf synthetischen Rohstoffen und entsprechen damit nicht dem wachsenden Bedürfnis des Marktes nach Produkten aus nachwachsenden Rohstoffquellen. Darüberhinaus neigen insbesondere die linearen Alkylbenzoate dazu, kunststoffhaltige Verpackungen anzugreifen, was eine Kontamination der Produkte durch Weichmacher oder ähnliche Stoffe nach sich ziehen kann.

Es wäre daher wünschenswert, Ölkomponenten zur Verfügung zu haben, welche für die oben genannten chemischen UV-Filter die geforderten technischen Anforderungen erfüllen, gleichermaßen jedoch ein angenehmes Hautgefühl erzeugen und darüber hinaus aus nachwachsenden Rohstoffen hergestellt und so den ökologischen Anforderungen der kosmetischen Industrie gerecht werden. Des weiteren wäre es hinsichtlich der Produktsicherheit vorteilhaft, ein Lösungsmittel zur Verfügung zu haben, welches sich hinsichtlich kunststoffhaltigen Verpackungsmaterialien inert verhält und möglichst preiswert ist.

Überraschenderweise und für den Experten nicht vorhersehbar wurde nun gefunden, dass mit dem Glyceryltriheptanoat (INCI: Triheptanoin) ein Triglycerid und Rohstoff aus nachwachsenden Quellen gefunden wurde, der
- ein gutes Lösungsvermögen für die UV-Filter Ethylhexyl Triazon, Butyl Methoxydibenzoylmethan und Bis-Ethylhexyloxyphenol Methoxyphenyl Triazine besitzt
- die Kristallisationsneigung der genannten UV-Filter auch in Gemischen mit anderen Ölkomponenten reduziert
- ein gutes Spreitvermögen besitzt und somit eine homogene Verteilung der genannten UV-Filter auf der Haut gewährleistet und ein angenehmes Hautgefühl erzeugt
- anorganische Pigmente gut dispergieren
- sich gegenüber Kunststoffverpackungen inert verhält und somit das Risiko einer Produktkontamination durch Weichmacher oder andere Bestandteile der Verpackung reduziert
und welcher damit den oben genannten technischen Anforderungen nicht nur für einen, sondern für alle genannten chemischen UV-Filter gerecht wird und den ökologischen und ökonomischen Nachteilen des Stands der Technik Abhilfe schafft, und somit die komplexe Aufgabenstellung in hervorragender Weise löst.

Das vorliegende Patent betrifft daher kosmetische und dermatologische Lichtschutzzubereitungen, insbesondere hautpflegende kosmetische und dermatologische Zubereitungen mit einem Gehalt an Triheptanoin und einen oder mehrere der UV-Filter 2,4,6-Trianilino-p-(carbo-2'-ethylhexyl-1'oxy)-1,3,5-triazin (Ethylhexyl Triazon), 1-(4-tert-butylphenyl)-3-(4-methoxyphenyl)-propan-1,3-dion (Butyl Methoxydibenzoylmethan) oder Bis-octyloxyphenol-methoxyphenyl-triazin (Bis-Ethylhexyloxyphenol Methoxyphenyl Triazine).

### Beschreibung der Erfindung

Glyceryltriheptanoat (Triheptanoin) findet vor allen im Nahrungsmittelbereich Anwendung. Dort wird es u.a. als Markierungsmittel für Fette verwendet, welches Butter zugesetzt wird, die zum subventionierten Export aus der Europäischen Union bestimmt ist, um einen illegalen Re-Import nachweisen zu können. In jüngerer Zeit wurden darüber hinaus vor allen in den USA weitere Anwendungsbereiche zur Behandlung von Stoffwechselkrankheiten des Fettsäureabbaus von im diätetischen Bereich erschlossen. So zeigen Patenten, die durch eine Enzymdefekt nicht in der Lage sind, gradzahlige Fettsäureketten zu metabolisieren, schwere Krankheitsbilder an den Muskeln und am Herzen, die durch eine diätetische Behandlung mit Triheptanoin signifikant gebessert werden können (C.R. Roe et al, J. Clin. Invest. 110, (2002) 259-269). Auch in kosmetischen Mitteln wurde der Einsatz von Triheptanoin schon beschrieben, ohne jedoch mit irgend einem Bezug zu Lichtschutzeigenschaften oder UV-Filtern.

Die Herstellung von Triheptanoin erfolgt durch Veresterung von Glycerin und Heptansäure. Da das eingesetzte Glycerin aus pflanzlichen Ölen und die Heptansäure aus Ricinusöl hergestellt werden können, erfüllt dieser Rohstoff somit die erfindungsgemäß geforderten Kriterien, aus erneuerbaren Rohstoffquellen zu stammen. Triheptanoin kann von der Dr. Straetmans GmbH unter dem Handelsnamen Dermofeel® TC-7 bezogen werden. Es war nun erstaunlich festzustellen, dass durch die erfindungsgemäße Zugabe von Triheptanoin zu kosmetischen Formulierungen, welche die oben genannten UV-Filter enthalten, eine vergleichbare Menge dieser UV-Filter gelöst werden konnte, wie mit den Butylene Glycol Diestern bzw. dem C12-15 Alkylbenzoaten. Im Falle des Ethylhexyl Triazon wurde die Menge gelösten UV-Filters sogar noch übertroffen.

In erfindungsgemäßen Formulierungen können die genannten UV-Filter alleine eingesetzt werden. Die Gesamtmenge an 2,4,6-Trianilino-p-(carbo-2'-ethylhexyl-1'oxy)-1,3,5-triazin (Ethylhexyl Triazon) wird dann vorteilhaft in einem Bereich von 0,1 % - 10 %, bevorzugt 0,5 - 6,0% gewählt, die Gesamtmenge an 1-(4-tert-butylphenyl)-3-(4-methoxyphenyl)-propan-1,3-dion (Butyl Methoxydibenzoylmethan) wird vorteilhaft im Bereich 0,1 - 6,0 % bevorzugt 0,5 - 4,0% gewählt, die Gesamtmenge an Bis-octyloxyphenol-methoxyphenyl-triazin (Bis-Ethylhexyloxyphenol Methoxyphenyl Triazine) wir vorteilhaft im Bereich 0,1 % - 15 %, bevorzugt 0,5 - 10 % gewählt. Die genannten UV-Filter können aber selbstverständlich auch beliebig miteinander kombiniert werden, um einen effektiven Breitbandschutz zu gewährleisten.

Die Gesamtmenge an Triheptanoin in erfindungsgemäßen Formulierungen beträgt 0,1 % - 50 %, vorzugsweise 0,5% - 15% bezogen auf das Gesamtgewicht der Formulierung. Es kann alleine aber selbstverständlich auch in Kombination mit anderen Ölkomponenten zum Lösen der genannten UV-Filter, wie z.B. C12-15 Alkylbenzoat oder Butylene Glycol Dicaprylat / Dicaprat verwendet werden. Die erfindungsgemäß hergestellten Formulierungen zeichnen sich durch ein überraschend ansprechendes Hautgefühl und eine gute Verteilung auf der Haut aus.

Die erfindungsgemäßen Sonnenschutzformulierungen können wie üblich zusammengesetzt sein und dem kosmetischen und/oder dermatologischen Lichtschutz aber auch zur Behandlung, Pflege und Reinigung der Haut und / oder Haare dienen oder auch als Produkt der dekorativen Kosmetik.

Sie können kosmetische Hilfsstoffe enthalten, wie sie üblicherweise in solchen Formulierungen verwendet werden wie beispielsweise Tenside, weitere Ölkörper, Emulgatoren, Perlglanzwachse, Konsistenzgeber, Verdickungsmittel, Überfettungsmittel, Stabilisatoren, Polymere, Siliconverbindungen Fette, Wachse, Lecithine, Phospholipide, biogene Wirkstoffe, UV-Lichtschutzfaktoren, Antioxidantien, Deodorantien, Antitranspirantien, Antischuppenmittel, Filmbildner, Quellmittel, Insektrepellentien, Selbstbräuner, Tyrosininhibitoren (Depigmentierungsmittel), Hydrotrope, Solubilisatoren, Konservierungsmittel, Parfümöle, Farbstoffe etc., die nachstehend exemplarisch aufgelistet sind.

### Tenside

Als oberflächenaktive Stoffe können anionische, nichtionische, kationische und/oder amphotere bzw. zwitterionische Tenside enthalten sein. In tensidhaltigen kosmetischen Zubereitungen, wie beispielsweise Duschgelen, Schaumbädern, Shampoos etc. ist vorzugsweise wenigstens ein anionisches Tensid enthalten. Der Anteil der Tenside liegt hier üblicherweise bei etwa 0,1 bis 30, vorzugsweise 5 bis 25 und insbesondere 10 bis 20 Gew.-%.

Typische Beispiele für anionische Tenside sind Seifen, Alkylbenzolsulfonate, Alkansulfonate, Olefinsulfonate, Alkylethersulfonate, Glycerinethersulfonate, □-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid (ether)sulfate, Mono- und Dialkylsulfosuccinate, Mono- und Dialkylsulfosuccinamate, Sulfotriglyceride, Amidseifen, Ethercarbonsäuren und deren Salze, Fettsäurethionate, Fettsäuresarcoinate, Fettsäuretauride, N-Acylaminosäuren, wie beispielsweise Acyllactylate, Acyltartrate, Acylglutamate und Acylaspartate, Alkyloligoglucosidulfate, Proteinfettsäurekondensate (insbesondere planzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettsäurepolyglycerylester, Fettalkoholpolyglycolether, Alkylphenolpolyglycolether, Fettsäurepolyglycolester, Fettsäureamidpolyglycolether, Fettaminpolyglyolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternisierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d. h. besonders hautverträgliche Tenside sind Fettalkoholpolyglycolethersulfate, Monoglyceridsulfate, Mono- und/oder Dialkylsulfosuccinate, Fettsäureisothionate, Fettsäuresarcosinate, Fettsäuretauride, Fettsäureglutamate, □-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen die sensorischen Eigenschaften weiter zu optimieren. Die Ölkörper sind üblicherweise in einer Gesamtmenge von 0,1 - 50 Gew.-%, vorzugsweise 5 - 25 Gew.-% und insbesondere 5 - 15 Gew.% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂₋Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, CVetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isosterylstearat, Isostearylisostearat, Isostearyloleat, Isostearylbehenat, Oleylmyristat, Oleylpalmitat, Oleylstearat, Oleylisostearat, Oleyloleat, Oleylbehenat, Oleylerucat, Behenylmyristat, Behenylpalmitat, Behenylpalmitat, Behenylstearat, Behenylisostearat, Behenyloleat, Behenylbehenat, Erucylmyristat, Erucylpalmitat, Erucylstearat, Erucylisostearat, Erucyloleat, Erucylbehenat und Erucylerucat in Betracht. Daneben eigenen sich auch Ester von linearen C₆₋C₂₂-Fettsäuren mit unverzweigten oder verzweigten mehrwertigen Alkoholen wie z.B. Butylene Glycol Dicaprylat/Dicaprat (Dermofeel® BGC) oder Ester des 2-Methyl-1,3-propandiols, des 1,1,1-Trimethylolethans oder 1,1,1-Trimethylolpropans. Des weiteren eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis von C₆-C₁₀₋Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z. B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Dicaprylyl Ether (Cetiol® OE), Ringöffnungsprodukte von epoxidierten Fettsäureestern mit Polyolen, Siliconöle (Cyclomethicone, Sliciummethicontypen u. a.) und/oder aliphatische bzw. naphthenische Kohlenwasserstoffe, wie z. B. Squalan, Squalen oder Dialkylcyclohexane.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/oder 0 bis 5 Mol Propylenoxid an linear Fettalkohole mit 8 bis 22C-Atomen, an Fettsäuren mit 1 bis 22 C-Atomen, an Alkylphenole mit 8 bis 15 C-Atomen in der Alkylgruppe sowie Alkylamine mit 8 bis 22 Kohlenstoffatomen im Alkylrest
- Alkyloligoglykoside mit 8 bis 22 Kohlenstoffatomen im Alkylrest und deren ethoxylierte Analoga
- Anlagerungsprodukte von 1 bis 15 Mol Ethylenoxid an Ricinusöl und/oder gehärtetes Ricinusöl
- Anlagerungsprodukte von 15 bis 60 Mol Ethylenoxid an Ricinusöl und/oder gehärtetes Ricinusöl
- Partialester von Glycerin und/oder Sorbitan mit ungesättigten, linearen oder gesättigten, verzweigten Fettsäuren mit 12 bis 22 Kohlenstoffatomenund/oder Hydroxyarbonsäuren mit 3 bis 18 Kohlenstoffatomen sowie deren Addukte mit 1 bis 30 Mol Ethylenoxid.
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z. B. Sorbit), Alkylglucosiden (z. B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin
- Mono-, Di- und Trialkylphosphate sowie Mono-, Di- und/oder Tri-PEG-alkylphosphate und deren Salze
- Wollwachsalkohole
- Polysiloxan-Polyalkyl-Polyether-Copolymere bzw. entsprechende Derivate
- Block-Copolymere z. B. Polyethylenglycol-30-Dipolyhydroxy-stearat
- Polymeremulgatoren, z. B. Pemulen-Typen (TR-1, TR-2) von Goodrich
- Polyalkylenglycole sowie
- Glycerincarbonat.

### - Ethylenoxidanlagerungsprodukte

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäure-mono- und diester von Anlagerungsprodukten von Ethylenoxid an Glycerin sind als Rückfettungsmittel für kosmetische Zubereitungen bekannt.

### - Sorbitanester

Als Sorbitanester kommen Sorbitanmonoisostearat, Sorbitansesquiisostearat, Sorbitandiisostearat, Sorbitantriisbstearat, Sorbitanmonooleat, Sorbitansequioleat, Sorbitandioleat, Sorbitantrioleat, Sorbitanmonoerucat, Sorbitansesquierucat, Sorbitandierucat, Sorbitantrierucat, Sorbitanmonoricinolat, Sorbitansesquiricinolat, Sorbitandiricinolat, Sorbitantriricinolat, Sorbitanmonohydroxystearat, Sorbitanmonotartrat, Sorbitansesquitartrat, Sorbitanditartrat, Sorbitantritartrat, Sorbitanmonocitrat, Sorbitansesquitartrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### - Polyglycerinester

Typische Beispiele für geeignet Polyglycerinester sind Polyglyceryl-3 Stearate (Dermofeel® PS), Polyglycerin-3-Palmitate (Dermofeel® PP), Polyglyceryl-6 Caprylate (Dermofeel® G 6 CY), Polyglyceryl-10 Laurate (Dermofeel® G 10 L), Polyglyceryl-2-Laurate (Dermofeel® G 2 L), Polyglyceryl-3-Laurate (Dermofeel® G 3 L), Polyglyceryl-5-Laurate (Dermofeel® G 5 L), Polyglyceryl-2 Dipolyhydroxystearate (Dehymuls® PGPH), Polyglycerin-3-Diisostearate (Lameform® TGI), Polyglyceryl-4 Isostearate (Isolan® Gl 34), Polyglyceryl-3 Methylglucose Distearate (Tego Care® 450), Polyglyceryl-3 Beeswax (Cera Bellina® ), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Dermofeel® PR) Polyglyceryl Dimerate Isostearate sowie deren Gemische. Beispiele für weitere geignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### - Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäure mit 12 bis 22 Kohlenstoffatomen, wie beispielsweise Azelainsäure oder Sebacinsäure.

### - Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-NN-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinate, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinate. Besonders geeignet ist das unter der CTFA-Bezeichnung Cocoamidopropyl Betaine bekannte Fettsäureamid-Derivat. Ebenfalls geeignete Emulgatoren sind ampholytische Tenside. Unter ampholyytischen Tensiden werden olche oberflächenaktiven Verbindungen verstanden, die außer einer C_{8/18}-Alkyl- oder Acylgruppe im Molekül mindestens eine freie Aminogruppe und mindestens eine -COOH oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren N-Alkyliminidipropionäuren, N-Hydroxyethyl-N-alkylamidopropylglycin, N-Alkyltaurine, N-Alkylsarcosine, 2-Alkylaminopropionsäuren und Alkylminoessigsäuren mit jeweilsetwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ des Esterquats, vorzugsweise methylquaternierte Difettsäuretriethanolaminester-Salze, besonders bevorzugt sind.

Bevorzugt im Sinne der Erfindung sind kosmetische und/oder pharmazeutische Zubereitungen, die
(a) 0,1 - 50 Gew.-% Triheptanoin
(b) 0,1 - 20 Gew.-% Tenside und/oder Emulgatoren und/oder Coemulgatoren
(c) 0,1 - 40 Gew.-% weiterer Ölkörper
(d) 0 - 98 Gew.-% Wasser
bezogen auf die Gesamtzusammensetzung enthalten.

### Fett und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d.h. feste oder flüssige, pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, - palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwchse), wie z. B. Montanesterwachse, Sasolwachse, hydrierte Jojobawachse sowie synthetische Wachse, wie z. B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher häufig auch als Phosphatidylcholine (PC) bezeichnet. Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierten Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 - 15 Kohlenstoffatomen und 2 bis 10 Hydroxylgruppen sowie deren Mischungen.

### Konsistenzgeber und Verdickungsmittel

Als weitere Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Bettracht. Bevorzugt ist eine Kombination dieser Stoffe mit Alkyloligoglucosiden und/ oder Fettsäure-N-methylglucamiden gleicher Kettenlänge und/ oder Polyglycerinpoly-12-hydroxystearaten. Geeignete Verdickungsmittel sind beispielsweise Aerosil-Typen (Hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und diester von Fettsäuren, Polyacrylate (z. B. Carbopole® und Permulene-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z. B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit geeigneter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z. B. Magnesium-, Aluminium- und/oder Zinkstearat bzw. ricinoleat eingesetzt werden.

### Polymere

Geeignete kationische Polymere sind beispielsweise kationische Cellulosederivate, wie z. B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinilimidazol-Polymere, wie z. B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z. B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlösliche Polymere, kationische Chitinderivate wie beispielsweise quaterniertes Chitosan, gegebenenfalls mikrokristallin verteilt, Kondensationsprodukte aus Dihalogenalkylen, wie z. B. Dibrombutan mit Bisdialkylaminen, wie z. B. Bis-Dimethylamino-1,3-propan, ktionischer Guar-Gum, wie z. B. Jaguar® CBS, Jaguar® C-17, Jaguar® C-16 der Firma Celanese, quaternierte Ammoniumsalz-Polymere, wie z. B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/ Maleinsäureanhydrid-Copolymere und deren Ester, unvernetzte und mit Polyolen vernetzte Polyacrylsäuren, Acrylamidopropyltrimethylammoniumchlorid/Acrylat-Copolymere, Octylacrylamid/-Methylmethacrylat/tert.-Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfilter und Antioxidantien

Neben den erfindungsgemäßen UV-Filtern können weitere UV-Filter in den erfindungsgemäßen Formulierungen vorliegen. UV-B-Filter können öllöslich oder wasserlöslich sein. Als öllösliche Substanzen sind z.B. zu nennen:
- 3-Benzylidencampher bzw. 3-3-Benzylidennorcampher und dessen Derivate, z.B. 3-(4-Methylbenzyliden)campher wie in der Patentschrift EP 0693471 B1 beschrieben.
- 4-Aminobenzoesäurederivate, vorzugsweise 4-(Dimethylamino)benzoesäure-2-ethylhexylester, 4-(Dimethylamino)benzoesäure-octylester und 4-(Dimethylamino)benzoesäure-2-amylester.
- Ester der Zimtsäure, vorzugsweise 4-Methoxyzimtsäure-2-ethylhexylester, 4-Methoxyzimtsäure-propylester, 4-Methoxyzimtsäure-isoamylester oder 2-Cyano-3,3-phenylzimtsäure-2-ethylhexylester (Octocrylene)
- Ester der Salicylsäure, vorzugsweise Salicylsäure-2-ethylhexylester, Salicylsäure-4-isopropylbenzylester oder Salicylsäurehomomenthylester
- Derivate des Bezophenons, vorzugsweise 2-Hydroxy-4-methoxybezophenon, 2-Hydroxy-4'-methoxybezophenon, 2,2'-Dihydoxy-4-methoxybenzophenon
- Ester der Benzalmalonsäure, vorzugsweise 4-Methoxybenzmalonsäure-di-2-ethylhexylester
- Triazinderivate wie z.B. Dioctyl Butamido Triazon (Uvasorb HEB)
- Ketotricyclo(5.2.1.0)decan-Derivate, wie in der EP 0694521 B1 beschrieben.

Als wasserlösliche Substanzen kommen in Frage:
- 2-Phenylbenzimidazol-5-sulfonsäure und deren Alkali-, Erdalkali-, Ammonium-, Alkylammonium, Alkanolammonium-und Glucammoniumsalze
- Sulfonsäurederivate von Benzophenon, vorzugsweise 2-Hydroxy-4-methoxybenzophenon-5-sulfonsäure und ihre Salze
- Sulfonsäurederivate des Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-Phenyl-3-(4'isopropylphenyl)-propan-1,3-dion sowie Enaminverbindungen, wie beschrieben in der DE 19712033 A1. Die UV-A- und UV-B-Filter können selbstverständlich auch in Mischungen eingesetzt werden. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium, Alkanolammonium- und Glucammonumsalze kombiniert.

Neben den genannten löslichen Stoffen kommen für diesen Zweck auch unlösliche Lichtschutzpigmente, nämlich feindisperse Metalloxide bzw. salze in Frage. Beispiele für geeignete Metalloxide sind insbesondere Titandioxid und Zinkoxid und daneben Oxide des Eisens, Zirkoniums, Siliziums, Mangans, Aluminiums und Cers sowie deren Gemische. Als salze können Silicate (Talk), Bariumsulfat oder Zinkstearat eingesetzt werden. Die Oxide und Salze werden in Form der Pigmente für hautpflegende und hautschützende Emulsionen du dekorative Kosmetik verwendet. Die Partikel sollen dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobisiert vorliegen. Typische Beispiele sind gecoatete Titandioxide, wie z.B. Titandioxid T 805 der Fa. Degussa oder Eusolex T 2000 der Fa. Merck. Als hydrophobe Coatingmittel kommen dabei vor allem Silicone und dabei speziell Trialkoxyoctylsilane oder Simethicone in Frage. In Sonnenschutzmitteln werden bevorzugt so genannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn schädliche UV-Strahlung in die Haut eindringt. Typische Beispiele sind hierfür Aminosäure(z.B. Histidin, Tyrosin oder Tryptophan) und deren Derivate, Imidazole (z.B. Urocaninsäure) und deren Derivate, Peptide wie D,L-Carnosin, D-Carnosin, L-Carnosin und deren Derivate, Carotinoide, Carotine (z.B. □-Carotin, □-Carotin, Lycopin) und deren Derivate, Liponsäuren und deren Derivate (z.B. Dihydroliponsäure), Aurothioglucose, Propylthiouracil und andere Thiole (z.B. Thioredoxin, Glutathion, Cystein, Cystin, Cysteamin un deren Glycolsyl-, N-Acetyl-, Mehyl-, Ethyl-, Propyl-, Amyl-, Butyl-, Lauryl-, Palmitoyl-, Oleyl-, □-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximin, Homocysteinsulfoximin, Butioninsulfoine, Penta-, Hexa-, Heptathioninsulfoximin) in sehr geringen verträglichen Dosierungen (z.B. pmol bis □mol), ferner Komplexbildener wie z.B. D-Hydroxyfettsäuren, Palmitinsäure, Phytinsäure, Lactoferrin), □-Hydroxysäurem (z.B. Zitronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. □-Linolensäure, Linolsäure, Ölsäure), Fol-säure und deren Derivate, Ubichinon und Ubichinol und deren Derivate, Vitamin C und Derivate (z.B. Ascorbyl Palmitat, Magnesium Ascorbylphosphat, Ascobylacetat), Tocopherole (z.B. □-Tocopherol, □-Tocopherol, □-Tocopheol □-Tocopherol) und deren Derivate (Tocopherly Acetat), Vitamin A und Derivate (Vitamin A Palmitat) soweie Koniferylbenzoat des Benzoeharzes, Rutinsäure und deren derivate, □-Glycosylrutin, Ferulasäure, Furfurylidengluticol, Carnosin, Butylhydrxytoluol, Butylhydroxyanisol, Nordihydroguarjakharzsäure, Nordihydroguarjaretsäure, Trihydroxybutyrophenon, Harnsäure und deren Derivate, Mannose und deren Derivate, Speroxid-Dismutase, Zink und dessen Derivate (ZnO, ZnSO₄), Selen un dessen Derivate (z.B. Selen-Methionin), Stilbene und deren Derivate (z.B. Stilbenoxid, trans-Stilbenoxid) und die errfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffn sind beispielsweise Tocopherol, Toco-pherolacetat, Tocopherolpalmitat, Ascorbinsäure, (Desoxy) Ribonucleinsäure und deren Fragmentierungsprodukte, □-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitaminkomplexe zu verstehen.

### Deodorantien und keimhemmende Mittel

Kosmetische Deodorantien (Desodorantien)wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### - Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethylphenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicyl säure-n-octylamid oder Salicylsäure-n-decylamid.

### - Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw. -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### - Geruchsabsorber

Als Geruchsabsorber eignen sich Stoffe, die geruchsbildende Verbindungen aufnehmen und weitgehend festhalten können. Sie senken den Partiladruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfumöle, die zusätzlich zu ihrer Funktion als Geruchsüberdecker den Deodorantien ihre jeweilige Duftnote verleihen. Als Parfumöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropinonat, und Benzylsalicylat. Zu den Ethern zähln beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten vrwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, □-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, □-Damascone, Geraniumöl Bourbon, Cyclohexylsalicylat, Vertofix Couer, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen eingesetzt.

### - Antitranspirantien

Antitranspirantien (Antiperspipantien) reduzieren durch Beeinflussung der Aktivität der ekkrinen Schweißdrüsen die Schweißbildung und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe
- Ölkomponenten
- nichtionische Emulgatoren
- Coemulgatoren
- Konsistenzgeber
- Hilfsstoffe wie z. B. Verdicker oder Komplexierungsmittel und/oder
- nichtässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat,Aluminiumchloridtartrat, Aluminium-Zirkonium-Trichlorohydrat, Aluminium-Zirkoniumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringen Mengen enthalten sein. Solche öllöslichen Hilfsmittel können z. B. sein:
- entzündungshemmende, hautschützende oder wohlriechende ätherische Öle
- synthetische hautschützende Wirkstoffe und/oder
- öllösliche Parfumöle

Übliche wasserlösliche Zusätze sind z. B. Konservierungsmittel, wasserlösliche Duftstoffe, pH-Wert-Stellmittel, z. B. Puffergemische, wasserlösliche Verdickungsmittel, z. B. wasserlösliche natürliche oder synthetische Polymere wie z. B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-VinylacetatCopolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimethylpentyl)-2-(1H)-pyridinonmonoethanolaminsalz), Baypival® (Climbazole), Ketoconazol®, (4-Acetyl-1-{-4-[2-(2.4-dichlorphenyl)-r-2-(1H-imidazol-1-ylmethyl)-1,3-dioxylan-c-4-ylmethoxyphenyl}piperazin, Elubiol, Selendisulfid, Schwefel kolloidal, Schwefelpolyethylenglykolsorbitanmonooleat, Schwefelricinolpolyethoxylat, Schwefel-Teer Destillate, Salicylsäure (bzw. in Kombination mit Hexachlorophen), Undexylensäure Monoethanolamid Sulfosuccinat Na-Salz, Lampeon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wässrige Phasen können Montmorillonite, Clay Mineralstoffe, Pemulen sowie alkyl-modifizierte Carbopoltypen (Goodrich) dienen. Weitere geeignete Polymere bzw. Quellmittel können der Übersicht von R. Lochhead in Cosm. Toil. 108, 95 (1993) entnommen werden.

### Insekt-Repellentien

Als Insekt-Repellentien kommen beispielsweise N,N-Diethyl-m-toluamid, 1,2-Pentandiol oder 3-(N-n-Butyl-N-acetyl-amino)-propionic aid ethyl ester), welches unter der Bezeichnung Insect Repellent® 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosininhibitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein.

Typische Beispiele sind:
- Glycerin
- Alkylenglycole, wie beispielsweise Ethylenglycol, Diethylenglycol, Propylenglycol, Butylenglycol, Hexylenglycol, 1,2-Hexandiol und Caprylyl Glycol sowie Polyethylenglycole mit einem durchschnittlichen Molekulargewicht von 100 bis 1.000 Dalton.
- technische Oligoglyceringemische mit einem Eigenkondensationsgrad von 1,5 bis 10 wie etwa technische Diglyceringemische mit einem Diglyceringehalt von 40 bis 50 Gew.-%
- Methylolverbindungen, wie insbesondere Trimethylolethan, Trimethylolpropan, Trimethylolbutan, Pentaerythrit und Dipentarythrit
- Niedriglkylglucoside, insbesondere solche mit 1 bis 8 Kohlenstoffen im Alkylrest, wie bespielsweise Methyl- und Butylglucosid
- Zuckeralkohole mit 5 bis 12 Kohlenstoffatomen, wie beispiele Sorbit oder Mannit
- Zucker mit 5 bis 12 Kohlenstoffatomen, wie beispielsweise Glucose oder Saccharose
- Aminozucker, wie beispielsweise Glucamin
- Dialkoholamine, wie Diethanolamin oder 2-Amino-1,3-propandiol

### Konservierungsmittel

Als Konservierungsmittel eignen sich beispielsweise Phenoxyethanol, Formaldehydlösung, Parabene, Pentandiol oder

Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle ein genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange),Wurzeln (Macis, Angelica, Sellerie, Kardamom, Costus, Iris, Calmus), Hölzern (Pinien- Sandel- Guajak-, Zedern-, Rosenholz), Kräutern und Grsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jojone, □-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydrmyrcenol, Lilial, Lyral, Citronellol, Phenylethylalkohol, □-Hexylzumtaldehyd, Geraniol, Benzylaceton, Cyclamenaldehyd, Linalool, Boisambrene Forte, Ambraxon, Indol, Hedione, Sandelice, Citronenöl, Mandarinenöl, Orangenöl, Allylamylglycolat, Cyclovertal, Lavandinöl, Muskateller Salbeiöl, □-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Farbstoffe

Als Farbtoffe können die für kosmetische Zwecke geeigneten und zugelassenen Substanzen verwendet werden. Beispiele sind Kochenillerot A (C.I. 16255), Patentblau V (C.I. 42051), Indigotin (C.I. 73015), Chlorophyllin (C.I. 75810), Chinolongelb (C.I. 47005), Titandioxid (C.I. 77891), Indanthrenblau RS (C.I. 69800) und Krapplack (C.I. 58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Die erfindungsgemäßen Formulierungen können dem Stand der Technik entsprechend hergestellt werden. Nachfolgend werden einige Beispiele genannt, die die vorliegende Erfindung verdeutlichen sollen, ohne diese einzuschränken.

| Sun Milk O/W | | |
|---|---|---|
| | | % |
| A | Wasser | Ad 100 |
| | Glycerin | 3,5 |
| | Konservierungsmittel | q.s |
| B | Glyceryl Stearate Citrate | 2,5 |
| | Triheptanoin | 12,0 |
| | Ethylhexyl Methoxycinnamate | 2,0 |
| | Butyl Methoxydibenzoylmethan | 2,0 |
| | Bis-Ethylhexyloxyphenol Methoxyphenyl Triazine | 1,0 |
| | Isoamyl Methoxycinnamate | 5,0 |
| | Glyceryl Stearate | 2,0 |
| | Cetearyl Alcohol | 1,0 |
| C | Sodium Carbomer | 0,2 |
| D | Parfum | q.s |

| Sun Milk W/O | | |
|---|---|---|
| | | % |
| A | Wasser | Ad 100 |
| | Glycerin | 2,0 |
| | Magnesium Sulfate | 0,7 |
| B | Triheptanoin | 12,0 |
| | Ethylhexyl Methoxycinnamate | 7,0 |
| | Butyl Methoxydibenzoylmethan | 1,0 |
| | Ethylhexyl Triazone | 2,0 |
| | PEG-30 Dipolyhydroxystearate | 3,0 |
| | Magnesium Stearate | 0,5 |
| | Tocopheryl Acetate | 1,0 |
| | Disteardimonium Hectorite | 0,3 |
| | DC 345 | 10,0 |
| | Konservierungsmittel | q.s. |
| | Parfum | q.s. |

## Patentansprüche

1. Kosmetische und dermatologische Lichtschutzzubereitungen enthaltend eine Wirkstoffkombination, bestehend aus einem oder mehreren UV-Filtern, der aus 2,9,6-Trianilino-p-(carbo-2'-ethylhexyl-1'oxy)-1,3,5-triazin (Ethylhexyl Triazon), 1-(4-tert-butylphenyl)-3-(4-methoxyphenyl)-propan-1,3-dion (Butyl Methoxydibenzoyl-methan), Bis-octyloxyphenol-methoxyphenyl-triazin (Bis-Ethylhexyloxyphenol Methoxyphenyl Triazine) bestehenden Gruppe und Triheptanoin.

2. Kombination gemäß Anspruch 1 **dadurch gekennzeichnet, dass** 2,4,6-Trianilino-p-(carbo-2'-ethylhexyl-1'oxy)-1,3,5-triazin (Ethylhexyl Triazon) in der fertigen kosmetischen oder dermatologischen Lichtschutzzubereitung in einer Konzentration von 0,1 % - 10 %, bevorzugt 0,5 % - 6,0 % bezogen auf das Gesamtgewicht der Formulierung enthaltend ist.

3. Kombination gemäß Anspruch 2 **dadurch gekennzeichnet, dass** Triheptanoin in der fertigen kosmetischen oder dermatologischen Lichtschutzzubereitung in einer Konzentration von 0,1 % - 50 %, bevorzugt 0,5 % - 15,0 % bezogen auf das Gesamtgewicht der Formulierung enthaltend ist.

4. Kombination gemäß Anspruch 1 **dadurch gekennzeichnet, dass** 1-(4-tert-butylphenyl)-3-(4-methoxyphenyl)-propan-1,3-dion (Butyl Methoxydibenzoylmethan) in der fertigen kosmetischen oder dermatologischen Lichtschutzzubereitung in einer Konzentration von 0,1 % - 6,0 %, bevorzugt 0,5 % - 9,0 % bezogen auf das Gesamtgewicht der Formulierung enthaltend ist.

5. Kombination gemäß Anspruch 4 **dadurch gekennzeichnet, dass** Triheptanoin in der fertigen kosmetischen oder dermatologischen Lichtschutzzubereitung in einer Konzentration von 0,1 % - 50 %, bevorzugt 0,5 % - 15,0 % bezogen auf das Gesamtgewicht der Formulierung enthaltend ist.

6. Kombination gemäß Anspruch 1 **dadurch gekennzeichnet, dass** Bis-octyloxyphenol-methoxyphenyl-triazin (Bis-Ethylhexyloxyphenol Methoxyphenyl Triazine) in der fertigen kosmetischen oder dermatologischen Lichtschutzzubereitung in einer Konzentration von 0,1 % - 15,0 %, bevorzugt 0,5 % - 10,0 % bezogen auf das Gesamtgewicht der Formulierung enthaltend ist.

7. Kombination gemäß Anspruch 6 **dadurch gekennzeichnet, dass** Triheptanoin in der fertigen kosmetischen oder dermatologischen Lichtschutzzubereitung in einer Konzentration von 0,1 % - 50 %, bevorzugt 0,5 % - 15,0 % bezogen auf das Gesamtgewicht der Formulierung enthaltend ist.

## Claims

1. Cosmetic and dermatological sunscreens containing an active agent combination comprising one or more UV filters, the group comprising 2,4,6-trianilino-p-(carbo-2'-ethylhexyl-1'oxy)-1,3,5-triazine (ethylhexyl triazone), 1-(4-tert-butylphenyl)-3-(4-methoxyphenyl)-propane-1,3-dione (butyl methoxydibenzoyl methane), bis-octyloxyphenol-methoxyphenyl-traizine (bis-ethylhexyloxyphenol methoxyphenyl triazine) and triheptanoin.

2. Combination according to claim 1, **characterized in that** 2,4,6-trianilino-p-(carbo-2'-ethylhexyl-1' oxy)-1,3,5-triazine (ethylhexyl triazone) is contained in the finished cosmetic or dermatological sunscreen in a concentration of 0.1 to 10%, preferably 0.5 to 6%, based on the total weight of the formulation.

3. Combination according to claim 1, **characterized in that** triheptanoin is contained in the finished cosmetic or dermatological sunscreen in a concentration of 0.1 to 50%, preferably 0.5 to 15%, based on the total weight of the formulation.

4. Combination according to claim 1, **characterized in that** 1-(4-tert-butylphenyl)-3-(4-methoxyphenyl)-propane-1,3-dione (butyl methoxydibenzoylmethane) is contained in the finished cosmetic or dermatological sunscreen in a concentration of 0.1 to 6%, preferably 0.5 to 4%, based on the total weight of the formulation.

5. Combination according to claim 1, **characterized in that** triheptanoin is contained in the finished cosmetic or dermatological sunscreen in a concentration of 0.1 to 50%, preferably 0.5 to 15%, based on the total weight of the formulation.

6. Combination according to claim 5, **characterized in that** bis-octyloxyphenol-methoxyphenyl-triazine (bis-ethylhexyloxyphenol methoxyphenyl triazine) is contained in the finished cosmetic or dermatological sunscreen in a concentration of 0.1 to 15%, preferably 0.5 to 10%, based on the total weight of the formulation.

7. Combination according to claim 5, **characterized in that** triheptanoin is contained in the finished cosmetic or dermatological sunscreen in a concentration of 0.1 to 50%, preferably 0.5 to 15%, based on the total weight of the formulation.

## Revendications

1. Ecrans solaires cosmétiques et dermatologiques, contenant une combinaison de substances actives constituée d'un ou plusieurs filtres à rayons ultraviolets du groupe comprenant la 2,4,6-trianilino-p-(carbo-2'-éthylhexyl-1'-oxy)-1,3,5-triazine (éthylhexyl-triazone), la 1-(4-tertio-butylphényl)-3-(4-méthoxyphényl)-propane-1,3-dione (butylméthoxydibenzoyl-méthane), la bis-octyloxyphénol-méthoxyphényltriazine (bis-éthylhexyloxyphénol-méthoxyphényl-triazine), et de triheptanoïne.

2. Combinaison suivant la revendication 1, **caractérisée en ce que** la 2,4,6-trianilino-p-(carbo-2'-éthylhexyl-1'-oxy)-1,3,5-triazine (éthylhexyl-triazone) est contenue dans l'écran solaire cosmétique ou dermatologique prêt à l'emploi à une concentration de 0,1 % - 10 %, avantageusement de 0,5 % - 6,0 % par rapport au poids total de la formulation.

3. Combinaison suivant la revendication 1, **caractérisée en ce que** la triheptanoïne est contenue dans l'écran solaire cosmétique ou dermatologique prêt à l'emploi à une concentration de 0,1 % - 50 %, avantageusement de 0,5 % - 15,0 % par rapport au poids total de la formulation.

4. Combinaison suivant la revendication 1, **caractérisée en ce que** la 1-(4-tertio-butylphényl)-3-(4-méthoxyphényl)-propane-1,3-dione (butyl-méthoxydibenzoyl-méthane) est contenue dans l'écran solaire cosmétique ou dermatologique prêt à l'emploi à une concentration de 0,1 % - 6,0 %, avantageusement de 0,5 % - 4,0 % par rapport au poids total de la formulation.

5. Combinaison suivant la revendication 1, **caractérisée en ce que** la triheptanoïne est contenue dans l'écran solaire cosmétique ou dermatologique prêt à l'emploi à une concentration de 0,1 % - 50 %, avantageusement de 0,5 % - 15,0 % par rapport au poids total de la formulation.

6. Combinaison suivant la revendication 5, **caractérisée en ce que** la bis-octyloxyphénol-méthoxyphényl-triazine (bis-éthylhexyloxyphénol-méthoxyphényl-triazine) est contenue dans l'écran solaire cosmétique ou dermatologique prêt à l'emploi à une concentration de 0,1 % - 15,0 %, avantageusement de 0,5 % - 10,0 % par rapport au poids total de la formulation.

7. Combinaison suivant la revendication 5, **caractérisée en ce que** la triheptanoïne est contenue dans l'écran solaire cosmétique ou dermatologique prêt à l'emploi à une concentration de 0,1 % - 50 %, avantageusement de 0,5 % - 15 % par rapport au poids total de la formulation.
